(19) Europäisches Patentamt

European Patent Office

Office européen des brevets

(11) **EP 0 794 802 B1**

(12) **FASCICULE DE BREVET EUROPEEN**

(45) Date de publication et mention
de la délivrance du brevet:
**27.02.2002 Bulletin 2002/09**

(21) Numéro de dépôt: **95940305.6**

(22) Date de dépôt: **13.11.1995**

(51) Int Cl.[7]: **A61K 51/12**, A61K 47/48

(86) Numéro de dépôt international:
**PCT/FR95/01489**

(87) Numéro de publication internationale:
**WO 96/14881 (23.05.1996 Gazette 1996/23)**

(54) **COMPLEXE D'INCLUSION D'UN ACIDE GRAS RADIOHALOGENE DANS UNE CYCLODEXTRINE**

INKLUSIONSKOMPLEXE VON RADIOHALOGENIERTEN FETTSÄUREN IN EINER ZYKLODEXTRINE

INCLUSION COMPLEX OF A RADIOHALOGENATED FATTY ACID IN A CYCLODEXTRIN

(84) Etats contractants désignés:
**AT BE CH DE DK ES FR GB IT LI NL SE**

(30) Priorité: **14.11.1994 FR 9413609**

(43) Date de publication de la demande:
**17.09.1997 Bulletin 1997/38**

(73) Titulaire: **CIS BIO INTERNATIONAL
91400 Saclay (FR)**

(72) Inventeurs:
 • **PASQUALINI, Roberto
  F-92140 Clamart (FR)**
 • **PERLY, Bruno
  F-78320 Le Mesnil-Saint-Denis (FR)**
 • **MAUCLAIRE, Laurent
  F-75013 Paris (FR)**
 • **DJEDAINI-PILARD, Florence
  F-91150 Etampes (FR)**
 • **MICHEL, Yves
  F-91400 Orsay (FR)**

(74) Mandataire: **Des Termes, Monique et al
Société Brevatome 3, rue du Docteur
Lancereaux
75008 Paris (FR)**

(56) Documents cités:
**WO-A-85/00753          WO-A-93/15765**

 • **DATABASE WPI Section Ch, Week 9506 Derwent
 Publications Ltd., London, GB; Class A96, AN
 95-041210 & JP,A,06 321 812 ( DAIICHI
 RADIOISOTOPE KENKYUSHO) , 22 Novembre
 1994**
 • **DATABASE WPI Section Ch, Week 8721 Derwent
 Publications Ltd., London, GB; Class B05, AN
 87-143566 & CA,A,1 221 105 ( TRIUMF) , 28 Avril
 1987**
 • **INTERNATIONAL JOURNAL OF RADIATION
 APPLICATIONS AND INSTRUMENTATION PART
 A: APPLIED RADIATION AND ISOTOPES, vol. 37,
 no. 8, EXETER GB, pages 777-788, H. J.
 MACHULLA ET AL. 'RADIOIODINATED FATTY
 ACIDS FOR CARDIOLOGICAL DIAGNOSIS.'**

## Description

La présente invention a pour objet la réalisation de compositions radiopharmaceutiques injectables contenant des acides gras radiohalogénés, destinées à l'étude des troubles du métabolisme cardiaque par scintigraphie.

L'étude fonctionnelle du coeur par voie externe permet d'apprécier d'éventuelles modifications pathologiques. Ainsi, l'image obtenue par injection du thallium-201 permet de visualiser la répartition du débit sanguin dans le myocarde, mais elle ne donne pas accès à l'état métabolique de la cellule myocardique.

Les acides gras à longue chaine constituent l'une des principales sources d'énergie du muscle cardiaque, puisqu'ils fournissent 65 % de l'énergie nécessaire au fonctionnement des cellules myocardiques. Comme la présence d'un halogène sur ces acides gras ne modifie en rien leurs propriétés métaboliques, le radiomarquage des acides gras par un halogène radioactif conduit donc à une excellente méthode d'exploration des troubles métaboliques des cellules myocardiques chez l'homme.

Actuellement, pour réaliser ces examens, on utilise des acides gras marqués à l'iode radioactif tels que l'acide 16-iodo($^{123}$I) 3-méthyl hexadécanoïque ($^{123}$IAGM). Dans les services de médecine nucléaire, on prépare ce produit juste avant l'injection par marquage de l'IAGM avec de l'iode-123. Ce traceur radiomarqué, insoluble dans les solvants aqueux, est ensuite dissous dans un liquide constitué par une solution de sérum-albumine humaine. L'emploi de sérum-albumine humaine pose certains problèmes en raison du contrôle très strict à exercer sur la qualité de la sérum albumine humaine afin d'éviter toute contamination virale au patient. Il serait donc particulièrement intéressant d'utiliser pour la solubilisation un composé ne présentant pas ces risques de contamination.

De plus, la préparation de l'acide gras marqué à l'iode-123 est une méthode assez lourde, qui nécessite du personnel qualifié et entraîné et un environnement stérile pour assurer l'innocuité du produit final.

Il serait donc intéressant pour améliorer la qualité du produit de pouvoir réaliser celui-ci dans un établissement pharmaceutique qui pourrait distribuer le produit radiomarqué prêt à l'emploi, stérilisé.

Cependant, lorsqu'on utilise de la sérum albumine humaine pour solubiliser le produit, une stérilisation par la chaleur est impossible car la sérum albumine est dégradée dans ces conditions.

La présente invention a précisément pour objet de remplacer la sérum albumine humaine par un produit ne présentant pas les inconvénients décrits ci-dessus, en vue d'obtenir une composition radiopharmaceutique d'acide gras radiohalogéné qui conduit de plus à une biodistribution améliorée de l'acide gras radiohalogéné.

Ce produit remplacant la sérum albumine humaine est une cyclodextrine ou l'un de ses dérivés.

Les cyclodextrines sont des oligosaccharides cycliques composés de 6($\alpha$), 7($\beta$) et 8 ($\gamma$) unités de D-glucopyranose reliées en $\alpha$-1,4. Dans ces cyclodextrines, la cavité interne hydrophobe est constituée des protons, alors que les groupements hydroxyle qui sont rejetés vers l'extérieur, engendrent une structure externe hydrophile. Cette cavité interne peut accueillir des molécules hydrophobes ou des parties de molécules organiques. Il se forme alors un complexe d'inclusion.

Cette capacité à inclure des molécules organiques a déjà été utilisée pour solubiliser des médicaments peu ou pas solubles dans l'eau, comme il est décrit dans US-A-4 727 064 et EP-A-0 149 197. Ainsi, des complexes d'inclusion ont été formés avec des composés organiques tels que les hormones, les stéroïdes, les glycosides cardiaques, les dérivés des benzodiazépines, les benzimidazoles, les pipéridines, les pipérazines, les imidazoles, etc.

Cependant, comme il est indiqué dans EP-A-0149197, la $\beta$-cyclodextrine et ses dérivés méthylés ne peuvent convenir pour une utilisation thérapeutique en raison de leur toxicité élevée.

Dans le domaine de la scintigraphie, WO-A-93/15765 décrit toutefois l'utilisation de cyclodextrines comme additif pour stabiliser certains constituants d'une trousse de produit radiopharmaceutique, notamment des ligands phosphorés comme la tris (3-méthoxypropyl)phosphine, contre l'oxydation et la volatilisation. Dans ce cas, il ne s'agit donc pas d'acides gras ; le radioélément est le technétium ou le rhénium dont la chimie est très différente de celle des halogénes utilisés dans l'invention ; et le but n'est pas de solubiliser le produit radiopharmaceutique, mais de stabiliser les constituants tels que les ligands phosphorés, d'une trousse de préparation d'un produit radiopharmaceutique.

La présente invention a précisément pour objet une composition radiopharmaceutique comprenant un acide gras radiohalogéné, dans laquelle l'acide gras est sous la forme d'un complexe d'inclusion avec une cyclodextrine, ce qui permet de solubiliser celui-ci dans une solution aqueuse sans ajouter de la sérum albumine humaine. Cette composition est utilisable pour l'examen scintigraphique du myocarde.

Les cyclodextrines utilisables dans cette composition peuvent être de différents types et comporter divers substituants. Elles sont choisies en fonction des propriétés que doit présenter ce complexe d'inclusion, en particulier comme produit radiopharmaceutique.

Aussi, on choisit une cyclodextrine résistant aux traitements de stérilisation utilisés et n'exerçant pas d'influence néfaste sur la biodistribution de l'acide gras.

De préférence, on utilise les $\beta$ et $\gamma$-cyclodextrines ou des dérivés mono ou disubstitués de celles-ci, en particulier, les dérivés alkylés ou hydroxyalkylés. De tels dérivés sont décrits par exemple dans le livre de Dominique Duchêne : "New Trends in Cyclodextrins and derivatives ; Ed. de Santé, 1991, pages 269 à 278, et 315 à 319. A titre d'exemple de tels dérivés, on peut citer l'heptakis-2,6-di-O-méthyl-cyclomaltoheptaose, dénommé ci-après 2,6-diméthyl β-cyclodextrine et l'heptakis-O-hydroxypropyl cyclomaltoheptaose dénommé ci-après hydroxypropyl β-cyclodextrine.

Les acides gras radiohalogénés utilisés dans le complexe d'inclusion peuvent être également de différents types. On peut utiliser en particulier des acides gras aliphatiques saturés ou insaturés, à chaîne linéaire ou ramifiée, comprenant de 14 à 18 atomes de carbone et des acides gras aromatiques comme les acides (iodophényl)alcanoïques, utilisables pour l'examen du myocarde.

L'halogène est généralement en position $\bar{\omega}$. Par ailleurs, lorsque l'acide gras est un acide aliphatique, il est avantageusement substitué en position β, par rapport au groupe carboxyle, par un ou plusieurs substituants alkyle, par exemple méthyle ou éthyle.

En effet, la métabolisation de l'acide gras par β-oxydation peut être ralentie par la présence en position β d'un ou deux méthyle. Le temps de rémanence de l'acide gras dans la cellule myocardique est ainsi augmenté, ce qui permet de réaliser l'examen scintigraphique avec plus de confort.

L'halogène radioactif de l'acide gras peut être choisi parmi les isotopes radioactifs de F, Cl, Br et I.

A titre d'exemples de tels isotopes, on peut citer $^{123}I$, $^{125}I$, $^{131}I$, $^{124}I$, $^{18}F$, $^{76}Br$, $^{82}Br$ et $^{34m}Cl$.

A titre d'exemples d'acides gras utilisables, on peut citer l'acide 16-iodo 3-méthyl hexadécanoïque, l'acide 16-iodo 3,3-diméthyl hexadécanoïque, l'acide 16-iodo 9-hexadécénoïque, l'acide (ortho iodophényl)pentadécanoïque, l'acide 15-(para-iodophényl)pentadécanoïque, et l'acide 15-(paraiodophényl)-3-méthyl pentadécanoïque.

L'emploi d'acides gras de ce type est décrit dans Eur. J. Nucl. Med., 1988, 14, p. 624-627 ; Nucl. Med. Biol. Vol. 17, n° 8, 1990, p. 745-749 et vol. 20, n° 6, 1993 p. 747-754 ; et Nuclear Medicine Communications, 1993, 14 p. 181-188 et 310-317.

De préférence, l'acide gras radiohalogéné est un acide 16-halogéno 3-méthyl hexadécanoïque, en particulier l'acide 16-iodo-3-méthyl hexadécanoïque (IAGM).

Le complexe d'inclusion utilisé dans les compositions radiopharmeutiques de l'invention peut être préparé par des méthodes classiques, par exemple par mélange d'une solution de l'acide gras radiohalogéné dans un solvant organique miscible à l'eau avec une solution aqueuse de la cyclodextrine utilisée, suivie d'une évaporation du solvant organique. On peut ensuite évaporer la solution aqueuse, lorsque l'on veut obtenir le complexe à l'état sec.

A titre d'exemple de solvant organique utilisable, on peut citer l'acétone.

Les quantités de cyclodextrine et d'acide gras radiohalogéné mises en présence doivent correspondre à un excès de cyclodextrine par rapport à l'acide gras. On peut utiliser, par exemple, des quantités d'acide gras radiohalogéné et de cyclodextrine telles que le rapport molaire cyclodextrine/acide gras radiohalogéné soit de 2 à 10. Généralement, de bons résultats sont obtenus avec un rapport molaire d'environ 5.

Selon une forme préférée de réalisation de l'invention, la composition radiopharmaceutique utilisable pour l'examen du myocarde, est constituée par une solution aqueuse du complexe d'inclusion de la cyclodextrine et de l'acide gras radiohalogéné.

L'acide gras radiohalogéné utilisé dans la composition radiopharmaceutique de l'invention, peut être préparé par un procédé classique, par exemple par le procédé décrit dans EP-A-0 069 648 lorsque l'halogène radioactif est $^{123}I$, $^{125}I$, $^{131}I$ ou $^{124}I$.

Pour obtenir la composition radiopharmaceutique, on reprend l'acide gras radiohalogéné, préalablement mis à sec, avec une solution aqueuse de cyclodextrine.

Après distribution en volumes de 2ml, chaque flacon peut être soumis à la stérilisation ; celle-ci peut être effectuée par des procédés classiques, par exemple par chauffage.

Dans la composition radiopharmaceutique, la solution aqueuse contient de préférence, d'autres additifs, en particulier pour amener l'osmolarité à une valeur adaptée à l'injection.

Les additifs utilisés dans ce but peuvent être des tampons, du sérum physiologique, et en particulier des polyols. A titre d'exemples de polyols utilisables, on peut citer le mannitol, l'inositol, le sorbitol et le dixylitol.

Ces additifs sont choisis en fonction des traitements éventuels que devra subir la solution, en particulier du mode de stérilisation envisagé.

Lorsque la stérilisation est effectuée par la chaleur, on utilise de préférence un polyol tel que le mannitol ou l'inositol.

L'invention a encore pour objet une trousse pour la préparation d'une composition radiopharmeutique à l'iode radioactif, à partir d'un acide gras non radioactif et de cyclodextrine.

Cette trousse comprend au moins deux flacons qui contiennent respectivement un seul ou plusieurs des constituants suivants :

- un acide gras bromé ou iodé, non radioactif,
- un solvant organique tel que l'acétone,
- un iodure métallique, tel que l'iodure de sodium,

- une cyclodextrine, et

- un excipient convenant à la préparation d'une solution injectable.

Avec cette trousse, on peut préparer tout d'abord l'acide gras marqué à l'iode radioactif en mettant en oeuvre le procédé décrit dans EP-A-0 069 648, à partir du ou des flacons contenant l'acide gras non radioactif, l'iodure métallique et le solvant organique, dont on combine le(s) contenu(s) avec de l'iodure radioactif.

Après cette opération, on mélange l'acide gras marqué à l'iode radioactif avec une solution aqueuse de cyclodextrine provenant d'un des flacons de la trousse

D'autres caractéristiques et avantages de l'invention apparaîtront mieux à la lecture des exemples suivants donnés bien entendu à titre illustratif et non limitatif, en référence aux figures 1 et 2 annexées.

La figure 1 représente le spectre obtenu par RMN du proton à 500 MHz du complexe d'inclusion acide 16-iodo-3-méthyl hexadécanoïque et 2,6-diméthyl β -cyclodextrine, et

La figure 2 représente le spectre RMN du proton à 500 MHz obtenu dans les mêmes conditions, de la 2,6-diméthyl β-cyclodextrine.

**Exemples 1 à 4 :** Solubilisation de l'acide 16-iodo 3-méthyl hexadécanoïque dans différentes cyclodextrines.

Dans ces exemples, on dissout 1 mg (2,5 µmol) d'acide 16-iodo 3-méthyl hexadécanoïque (IAGM) dans 500 µl d'acétone. Après évaporation partielle de l'acétone, on ajoute un équivalent de la cyclodextrine utilisée (2,5 µmol) dissous dans 500 µl de solution tampon phosphate 0,1M, pH de 7,4. On agite vigoureusement en chauffant. Après refroidissement, on regarde l'évolution de la solution. Lorsqu'elle reste inchangée dans le temps, la limite de solubilité est atteinte. Dans le cas contraire, on ajoute 1, puis 2, puis 3 équivalents, etc. pour obtenir la solution désirée.

Les résultats obtenus avec différents cyclodextrines sont rassemblés dans le tableau 1 qui suit.

Dans ce tableau, on a indiqué les équivalents de cyclodextrine nécessaires pour obtenir une solution limpide.

Les résultats du tableau 1 montrent que la 2,6-diméthyl β-cyclodextrine et la 2-hydroxypropyl β-cyclodextrine fournissent les meilleurs résultats. De bons résultats sont obtenus également avec la γ-cyclodextrine. En revanche, on n'obtient pas de complexe avec la 2,3,6-triméthyl β-cyclodextrine.

On étudie par résonance magnétique nucléaire (RMN) du proton à 500 MHz le complexe obtenu avec la 2,6-diméthyl β-cyclodextrine. Le spectre obtenu avec le complexe d'inclusion dissous à une concentration de 4 mmol/l dans la solution tampon phosphate à 298 K est représenté sur la figure 1.

Sur la figure 2, on a représenté à titre comparatif le spectre RMN du proton à 500 MHz de la 2,6-diméthyl β-cyclodextrine seule à une concentration de 10 mmol/l dans le même tampon phosphate.

La comparaison de ces deux spectres montre que l'on a bien produit un complexe d'inclusion, car on constate un déplacement des protons $H^3$ et $H^5$ vers les champs forts ; ainsi les protons situés dans la cavité interne sont perturbés par le composé inclus. L'inclusion de l'acide gras se fait par l'halogène suivi de quelques $CH_2$.

Une étude en milieu diméthylsulfoxyde (DMSOd6) a permis de mettre en évidence une stoechiométrie 1/1 pour le complexe et une constante d'association de 500.

**Exemples 5 à 14** : Stabilité de différents complexes à la stérilisation par chauffage.

Dans ces exemples, on prépare différents complexes de cyclodextrine avec l'acide 16-iodo 3-méthyl hexadécanoïque (IAGM) en opérant de la façon suivante. Dans un flacon, contenant 2,02 µmol de IAGM on ajoute 10,1 µmol de la cyclodextrine utilisée, mentionnée dans le Tableau 2 qui suit, dissoute dans 2 ml du solvant utilisé qui est donné également dans le Tableau 2. On soumet la solution à une stérilisation par la chaleur dans les conditions données dans le Tableau 2.

On détermine la pureté radiochimique du complexe avant stérilisation et après stérilisation. Cette pureté radiochimique (en %) correspond au pourcentage de radioactivité présent dans le complexe par rapport à la radioactivité totale.

Les résultats sont donnés dans le Tableau 2.

Au vu des ces résultats, on remarque que le solvant de dissolution de la cyclodextrine a une grande importance. En effet, en présence d'ions chlorure ou carbonates, on a une pureté radiochimique beaucoup plus faible après stérilisation, ce qui n'est pas le cas avec l'eau. Les meilleurs solvants sont l'eau et les solutions de mannitol ou d'inositol à 4 % dans l'eau. La pression osmotique de ces dernières étant comprise entre 280 et 300 mOsm/kg, ces solutions peuvent être utilisées pour la fabrication de produits radiopharmaceutiques injectables.

**Exemple 15** : Etude de la fixation cardiaque du complexe d'inclusion d'acide 16-iodo 3-méthyl hexadécanoïque (IAGM) avec la 2,6-diméthyl-β -cyclodextrine.

Dans cet exemple, on injecte à des chiens Beagle pesant 15 à 18 kg, 2 ml d'une solution aqueuse de complexe d'inclusion obtenue par dissolution de 0,8 mg d'acide 16-iodo 3-méthyl hexadécanoïque marqué l'iode 123 (74 MBq) dans une solution d'inositol à 4 dans l'eau (ppi) contenant 14 mg de 2,6-diméthyl-β -cyclodextrine.

La solution aqueuse est préparée en suivant le mode opératoire de l'exemple 5 puis stérilisée par chauffage à 120°C pendant 20 min.

L'acide gras marqué à l'iode 123 a été préparé de la façon suivante :

8mg d'acide gras sont dissous dans une solution acétonique contenant 10 μg d'iode-127. La solution est ajoutée à la solution d'iode-123 (1850 MBq à calibration). L'ensemble est porté pendant 7 minutes à 100°C. Après refroidissement, la solution est purifiée sur la résine anionique AG 1 x 8 100-200 mesh. la solution acétonique récupérée est mise à sec. Le résidu solide est repris par 20 ml d'une solution de mannitol ou d'inositol à 4% contenant 144 mg de 2,6-diméthyl-β-cyclodextrine. Après dissolution totale, la solution est répartie en flacons (2 ml par flacon). Chaque flacon est serti et soumis à la stérilisation par chauffage.

Après l'injection, on examine les chiens au moyen d'une caméra en prenant une image par minute pour déterminer la captation et la rétention de l'acide gras marqué à l'iode 123.

Les résultats obtenus sont donnés dans le tableau 3 annexé.

Ils sont estimés à partir de valeurs chiffrées, déterminées à partir des images, en utilisant le mode de calcul décrit dans Eur. J. Nucl. Med., 1988, 14 : p.624-627.

Dans ce tableau, l'indice de captation est le rapport entre la radioactivité présente dans le coeur à 2 min. (en cpm/pixel) sur l'activité circulante (en cpm/pixel).

Les pourcentages de rétention à 30 minutes et à 60 minutes correspondent au rapport entre la radioactivité cardiaque (après soustraction de l'activité circulante) à 30 minutes ou à 60 minutes et la radioactivité cardiaque à 10 minutes.

**Exemple 16 :**

On suit le même mode opératoire que dans l'exemple 15 pour étudier la fixation cardiaque du même complexe d'inclusion en remplaçant la solution aqueuse d'inositol par une solution de mannitol à 4 % dans l'eau ppi.

Les résultats obtenus sont également donnés dans le tableau 3.

Dans ce tableau, on a donné de plus à titre comparatif les résultats obtenus lorsqu'on injecte aux chiens 5 ml d'une solution d'acide 16-iodo 3-méthyl hexadécanoïque (8 mg) marqué à l'iode 123 (74MBq) dissous dans un tampon carbonate/hydrogénocarbonate pH=9 avec de la sérum albumine humaine.

Les résultats du tableau 3 montrent que l'on obtient une bonne fixation cardiaque.

Par ailleurs, on a remarqué sur les images que la fixation hépatique, 15 à 20 minutes après l'injection, est plus faible avec les solutions de l'invention que celle obtenue avec la solution de sérum albumine humaine.

Ainsi, l'emploi de la cyclodextrine permet non seulement d'éviter les risques de contamination virale, mais aussi d'améliorer la biodistribution de l'acide gras.

TABLEAU 1

| Ex. | Cyclodextrine | Equivalents de cyclodextrine/masse d'acide gras | Observations |
|---|---|---|---|
| 1 | 2,6-diméthyl β cyclodextrine | 5 éq./16,6mg | solution limpide incolore |
| 2 | 2,3,6-triméthyl β cyclodextrine | - | solution continuellement opalescente |
| 3 | 2-hydroxypropyl β cyclodextrine | 5 éq./21,3mg | solution limpide incolore |
| 4 | γ-cyclodextrine | 7 éq./24,9mg | solution limpide |

EP 0 794 802 B1

## TABLEAU 2

| EX | Cyclodextrine | Stérilisation | Solvant de la cyclodextrine | Pureté radiochimique (%) | |
|---|---|---|---|---|---|
| | | | | avant stérilisation | après stérilisation |
| 5 | 2-hydroxypropyl β-cyclodextrine | 110°C 30 min | Tampon $CO_3^-/HCO_3^-$ aqueux pH=9 | 92,5 | 75,8 |
| 6 | 2-hydroxypropyl β-cyclodextrine | 110°C 30 min | Tampon phosphate aqueux pH=7,4 | 97,2 | 91,3 |
| 7 | 2-hydroxypropy β-cyclodextrine | 110°C 30 min | 0,5ml tampon phosphate aqueux + 0,5 ml tampon phosphate en sérum physiologique pH=7,4 | 91,4 | 69,2 |
| 8 | 2,6-diméhtyl β-cyclodextrine | 110°C 30 min | $H_2O$ | 92,6 | 86,7 |
| 9 | 2,6-diméthyl β-cyclodextrine | 120°C 20 min | $H_2O$ | 99,1 | 98 |
| 10 | 2,6-diméthyl β-cyclodextrine | 120°C 20 min | 1ml $H_2O$ + 1ml sérum physiologique | 99,4 | 91,7 |
| 11 | 2,6-diméthyl β-cyclodextrine | 120°C 20 min | $H_2O$ | 99,3 | 97,2 |
| 12 | 2,6-diméthyl β-cyclodextrine | 120°C 20 min | Solution à 4 % en inositol | 92,8 | 88,7 |
| 13 | 2,6-diméthyl β-cyclodextrine | 120°C 20 min | Solution à 4 % en mannitol | 99,8 | 96,1 |
| 14 | 2-hydroxypropyl γ-cyclodextrine | 110°C 30 min | $H_2O$ | 97,6 | 91,2 |

## TABLEAU 3

| EXEMPLES | EXEMPLE COMPARATIF | 15 | 16 |
|---|---|---|---|
| Produit injecté | $\underline{IAGM}$ avec HSA | $\underline{IAGM}$ avec inositol | $\underline{IAGM}$ avec mannitol |
| Indice de captation | 0,54 | 0,55 | 0,75 |
| Rétention à 30 min (%) | 70 | 71 | 74 |
| Rétention à 60 min (%) | 69 | 55 | 64 |

EP 0 794 802 B1

**Revendications**

1. Composition radiopharmaceutique comprenant un acide gras radiohalogéné, **caractérisé en ce que** l'acide gras radiohalogéné est sous la forme d'un complexe d'inclusion avec une cyclodextrine.

2. Composition radiopharmaceutique selon la revendication 1, **caractérisée en ce que** la cyclodextrine est une β-cyclodextrine, une γ-cyclodextrine ou un dérivé de celles-ci.

3. Composition selon la revendication 2, **caractérisée en ce que** la cyclodextrine est l'heptakis-2,6-di-O-méthyl-cyclo-maltoheptaose.

4. Composition selon la revendication 2, **caractérisée en ce que** la cyclodextrine est l'heptakis-O-hydoxypropyl cyclomaltoheptaose.

5. Composition selon l'une quelconque des revendications 1 à 4, **caractérisée en ce que** l'acide gras est un acide gras ω-radiohalogéné aliphatique comprenant de 14 à 18 atomes de carbone, qui est éventuellement mono ou disubstitué en position β par un ou deux groupes alkyle, ou un acide gras aromatique.

6. Composition selon l'une quelconque des revendications 1 à 5, **caractérisée en ce que** l'halogène radioactif de l'acide gras est choisi parmi les isotopes radioactifs de F, Cl, Br et I.

7. Composition selon la revendication 6, **caractérisée en ce que** l'halogène est $^{123}$I, $^{125}$I, $^{131}$I ou $^{124}$I.

8. Composition selon l'une quelconque des revendications 1 à 7, **caractérisée en ce que** l'acide gras est l'acide 16-iodo 3-méthyl hexadécanoïque.

9. Composition radiopharmaceutique selon l'une quelconque des revendications 1 à 8, **caractérisée en ce qu'**elle est constituée par une solution aqueuse du complexe d'inclusion de la cyclodextrine et de l'acide gras radiohalogéné.

10. Composition radiopharmaceutique selon la revendication 9, **caractérisée en ce qu'**elle comprend au moins un autre additif pour amener l'osmolarité de la solution aqueuse à une valeur adaptée à l'injection.

11. Composition radiopharmaceutique selon la revendication 10, **caractérisée en ce que** l'additif est l'inositol ou le mannitol.

12. Procédé de préparation d'une composition radiopharmaceutique selon l'une quelconque des revendications 1 à 11, **caractérisé en ce qu'**il consiste à mélanger l'acide gras radiohalogéné amené à sec avec une solution aqueuse de cyclodextrine.

13. Procédé selon la revendication 12, **caractérisé en ce que** l'on soumet la solution obtenue à une stérilisation par chauffage.

14. Procédé selon l'une quelconque des revendications 12 et 13, **caractérisé en ce qu'**on utilise des quantités d'acide gras radiohalogéné et de cyclodextrine telles que le rapport molaire de la cyclodextrine à l'acide gras radiohalogéné soit de 2 à 10.

15. Procédé selon l'une quelconque des revendications 12 et 14, **caractérisé en ce que** la solution aqueuse de cyclodextrine comprend un additif choisi parmi les tampons et les polyols pour régler l'osmolarité de la solution.

16. Procédé selon la revendication 13, **caractérisé en ce que** l'additif est un polyol.

17. Procédé selon la revendication 16, **caractérisé en ce que** l'additif est l'inositol ou le mannitol.

18. Trousse pour la préparation d'une composition radiopharmaceutique à l'iode radioactif, selon l'une quelconque des revendications 1 à 11, **caractérisée en ce qu'**elle comprend au moins deux flacons qui contiennent respectivement un seul ou plusieurs des constituants suivants:

- un acide gras iodé ou bromé non radioactif,
- un solvant organique,
- un iodure métallique,
- une cyclodextrine, et
- un excipient convenant à la préparation d'une solution injectable.

**Claims**

1. Radiopharmaceutical composition including a radiohalogenated fatty acid, **characterized in that** the radiohalogenated fatty acid is in the form of an inclusion complex with a cyclodextrin.

2. Radiopharmaceutical composition according to Claim 1, **characterized in that** the cyclodextrin is a β-cyclodextrin, a γ-cyclodextrin or a derivative of them.

3. Composition according to Claim 2, **characterized in that** the cyclodextrin is heptakis(2,6-di-O-methyl)cyclomaltoheptaose.

4. Composition according to Claim 2, **characterized in that** the cyclodextrin is heptakis(O-hydroxy-propyl)cyclomaltoheptaose.

5. Composition according to any one of Claims 1 to 4, **characterized in that** the fatty acid is an ω-radiohalogenated aliphatic fatty acid that includes 14 to 18 carbon atoms, which is possibly mono- or disubstituted in the β position by one or two alkyl groups, or an aromatic fatty acid.

6. Composition according to any one of Claims 1 to 5, **characterized in that** the radioactive halogen of the fatty acid is chosen from among the radioactive isotopes of F, Cl, Br and I.

7. Composition according to Claim 6, **characterized in that** the halogen is $^{123}$I, $^{125}$I, $^{131}$I or $^{124}$I.

8. Composition according to any one of Claims 1 to 7, **characterized in that** the fatty acid is 16-iodo-3-methylhexadecanoic acid.

9. Radiopharmaceutical composition according to any one of Claims 1 to 8, **characterized in that** it comprises an aqueous solution of the inclusion complex of the cyclodextrin and the radiohalogenated fatty acid.

10. Radiopharmaceutical composition according to Claim 9, **characterized in that** it includes at least one other additive to bring the osmolarity of the aqueous solution to a value suitable for injection.

11. Radiopharmaceutical composition according to Claim 10, **characterized in that** the additive is inositol or mannitol.

12. Method for the preparation of a radiopharmaceutical composition according to any one of Claims 1 to 11, **characterized in that** it consists in mixing the radiohalogenated fatty acid reduced to dryness with an aqueous solution of cyclodextrin.

13. Method according to Claim 12, **characterized in that** the solution obtained is subjected to sterilization by heating.

14. Method according to either one of Claims 12 and 13, **characterized in that** quantities of radiohalogenated fatty acid and of cyclodextrin are used such that the molar ratio of the cyclodextrin to the radiohalogenated fatty acid is from 2 to 10.

15. Method according to either one of Claims 12 and 14, **characterized in that** the aqueous solution of cyclodextrin includes an additive chosen from among the buffers and the polyols in order to adjust the osmolarity of the solution.

16. Method according to Claim 13, **characterized in that** the additive is a polyol.

17. Method according to Claim 16, **characterized in that** the additive is inositol or mannitol.

**18.** Kit for the preparation of a radiopharmaceutical composition with radioactive iodine, according to any one of Claims 1 to 11, **characterized in that** it includes at least two flasks which contain respectively just one or more than one of the following constituents:

- a non-radioactive iodinated or brominated fatty acid,
- an organic solvent,
- a metallic iodide,
- a cyclodextrin, and
- an excipient suitable for the preparation of an injectable solution.


**Patentansprüche**

**1.** Radiopharmazeutische Zusammensetzung, enthaltend eine Radiohalogenfettsäure, **dadurch gekennzeichnet, daß** die Radiohalogenfettsäure in Form eines Einschlußkomplexes mit einem Cyclodextrin vorliegt.

**2.** Radiopharmazeutische Zusammensetzung nach Anspruch 1, **dadurch gekennzeichnet, daß** es sich bei dem Cyclodextrin um ein β-Cyclodextrin, ein γ-Cyclodextrin oder ein Derivat davon handelt.

**3.** Zusammensetzung nach Anspruch 2, **dadurch gekennzeichnet, daß** es sich bei dem Cyclodextrin um Heptakis-2,6-di-O-methyl-cyclomaltoheptaose handelt.

**4.** Zusammensetzung nach Anspruch 2, **dadurch gekennzeichnet, daß** es sich bei dem Cyclodextrin um Heptakis-O-hydroxypropyl-cyclomaltoheptaose handelt.

**5.** Zusammensetzung nach einem der Ansprüche 1 bis 4, **dadurch gekennzeichnet, daß** es sich bei der Fettsäure um eine aliphatische ω-Radiohalogenfettsäure mit 14 bis 18 Kohlenstoffatomen, die gegebenenfalls in β-Position durch eine oder zwei Alkylgruppen mono-oder disubstituiert ist, oder eine aromatische Fettsäure handelt.

**6.** Zusammensetzung nach einem der Ansprüche 1 bis 5, **dadurch gekennzeichnet, daß** das radioaktive Halogen der Fettsäure unter den radioaktiven Isotopen von F, Cl, Br und I ausgewählt ist.

**7.** Zusammensetzung nach Anspruch 6, **dadurch gekennzeichnet, daß** es sich bei dem Halogen um $^{123}$I, $^{125}$I, $^{131}$I oder $^{124}$I handelt.

**8.** Zusammensetzung nach einem der Ansprüche 1 bis 7, **dadurch gekennzeichnet, daß** es sich bei der Fettsäure um 16-Iod-3-methylhexadecansäure handelt.

**9.** Radiopharmazeutische Zusammensetzung nach einem der Ansprüche 1 bis 8, **dadurch gekennzeichnet, daß** sie aus einer wäßrigen Lösung des Einschlußkomplexes aus dem Cyclodextrin und der Radiohalogenfettsäure besteht.

**10.** Radiopharmazeutische Zusammensetzung nach Anspruch 9, **dadurch gekennzeichnet, daß** sie mindestens ein weiteres Additiv zur Einstellung der Osmolarität der wäßrigen Lösung auf einen für die Injektion geeigneten Wert enthält.

**11.** Radiopharmazeutische Zusammensetzung nach Anspruch 10, **dadurch gekennzeichnet, daß** es sich bei dem Additiv um Inosit oder Mannit handelt.

**12.** Verfahren zur Herstellung einer radiopharmazeutischen Zusammensetzung nach einem der Ansprüche 1 bis 11, **dadurch gekennzeichnet, daß** man die getrocknete Radiohalogenfettsäure mit einer wäßrigen Cyclodextrinlösung mischt.

**13.** Verfahren nach Anspruch 12, **dadurch gekennzeichnet, daß** man die erhaltene Lösung einer Hitzesterilisation unterwirft.

**14.** Verfahren nach Anspruch 12 oder 13, **dadurch gekennzeichnet, daß** man solche Mengen von Radiohalogenfettsäure und Cyclodextrin einsetzt, daß das Molverhältnis von Cyclodextrin zu Radiohalogenfettsäure 2 bis 10 beträgt.

**15.** Verfahren nach Anspruch 12 oder 14, **dadurch gekennzeichnet, daß** die wäßrige Cyclodextrinlösung ein unter Puffern und Polyolen ausgewähltes Additiv zur Regulierung der Osmolarität der Lösung enthält.

**16.** Verfahren nach Anspruch 15, **dadurch gekennzeichnet, daß** es sich bei dem Additiv um ein Polyol handelt.

**17.** Verfahren nach Anspruch 16, **dadurch gekennzeichnet, daß** es sich bei dem Additiv um Inosit oder Mannit handelt.

**18.** Kit zur Herstellung einer radiopharmazeutischen Zusammensetzung mit radioaktivem Iod nach einem der Ansprüche 1 bis 11, **dadurch gekennzeichnet, daß** es mindestens zwei Flaschen umfaßt, die jeweils einen oder mehrere der folgenden Bestandteile enthalten:

- eine nicht radioaktive Iod- oder Bromfettsäure,
- ein organisches Lösungsmittel,
- ein Metalliodid,
- ein Cyclodextrin und
- einen zur Herstellung einer Injektionslösung geeigneten Trägerstoff.